Europäisches Patentamt

European Patent Office

Office européen des brevets

(19)

(11) Numéro de publication : **0 006 856**
**B1**

(12) # FASCICULE DE BREVET EUROPÉEN

(45) Date de publication du fascicule du brevet :
09.09.81

(21) Numéro de dépôt : 78900014.8

(22) Date de dépôt : 01.06.78

(86) Numéro de dépôt international :
PCT/CH 78/00001

(87) Numéro de publication internationale :
WO WO/78000 (21.12.78 Gazettee 78/09)

(51) Int. Cl.³ : **A 23 K   1/22, C 07 H  13/12**

(54) **Aliment pour betail et procédé de préparation de cet aliment.**

(30) Priorité : 10.06.77 CH 7159/77

(43) Date de publication de la demande :
23.01.80 (Bulletin 80/02)

(45) Mention de la délivrance du brevet :
09.09.81 Bulletin 81/36

(84) Etats contractants désignés :
CH DE FR GB LU SE

(56) Documents cités :
DE - C - 727 691
US - A - 2 612 497
US - A - 2 687 354

Advances in Carbohydrate Chemistry, Vol. 13
(1958), Melville L. Wolfrom and R. Stuart Tipson,
Editors. Academic Press Inc., NEW YORK
I. GOODMAN « Glycosyl Ureides »
Pages 215-236

Chemical Abstracts, Vol. 83, N° 25, publié le 22
décembre 1975, COLUMBUS, OHIO, USA
L. N. GALYAMIN « Glucosylureide preparation in
the rations of calves »
Voir page 303, colonne 2, abrégé 205105y, Zhivotnovodstvo 1975, (9), 30-1 (Russ.)

Chemical Abstracts, Vol. 87, N° 3, publié le 18
juillet 1977, COLUMBUS, OHIO, USA
A. F. GLAZOV et al. « Urea, diammonium phosphate, and glucosylurea feeding of young cattle »
Voir page 481, colonne 1, abrégé n° 21065m. Rol
Uglekistoty Ammonilnogo Azota Protsessakh
Biosinth. Mater. Vses. Simp., 1976, 128-31 (Russ.)

(73) Titulaire : **BATTELLE MEMORIAL INSTITUTE**
7 route de Drize
CH-1227 Carouge/Genève (CH)
GB SE
SOLAGRO S.A.
Rue du Prince Albert 33
B-1050 Bruxelles (BE)
CH DE FR LU

(72) Inventeur : REGNAULT, Alain
Villard-Tacon
F-01210 Par Ornex (FR)
Inventeur : SACHETTO, Jean-Pierre
L'Escalade, Bat. D2
F-74160 Saint-Julien-en-Genevois (FR)

(74) Mandataire : Dousse, Blasco et al
7, route de Drize
CH-1227 Carouge/Genève (CH)

Imprimerie Jouve, 17, rue du Louvre, 75001 Paris, France

## Aliment pour bétail et procédé de préparation de cet aliment

### Domaine technique

La présente invention concerne un aliment pour le bétail à base d'hydrates de carbone et d'un dérivé d'urée.

L'invention concerne également un procédé de fabrication de cet aliment et, notamment, du dérivé d'urée qui en fait partie.

L'aliment suivant l'invention conduit, à la fois, à une assimilation plus favorable de l'azote combiné dans l'urée et à une amélioration de celle des hydrates de carbone présents dans l'aliment, ceci en comparaison avec les produits existants.

### Technique antérieure

On sait que pour croître normalement, les ruminants ont besoin, comme les autres êtres vivants, d'une alimentation équilibrée comportant, notamment, des proportions convenables d'hydrates de carbone et de matières azotées. On sait aussi que ces animaux sont capables de digérer et d'assimiler une proportion notable des hydrates de carbone se présentant sous forme de produits cellulosiques.

Par ailleurs, grâce à la présence de certains microorganismes dans leur tube digestif, les ruminants sont capables de synthétiser, à partir d'ammoniaque et de sucres, les acides aminés nécessaires à la constitution de leurs tissus organiques. Cet ammoniaque peut être engendré, dans la panse des ruminants, par l'hydrolyse enzymatique de composés azotés, notamment d'urée distribuée dans le fourrage. Cependant, la vitesse de formation de cet ammoniaque à partir d'urée est, dans l'estomac des ruminants, beaucoup plus élevée que celle de son utilisation par les microorganismes pour la synthèse des protéines génératrices desdits acides aminés. En conséquence, l'utilisation d'urée dans le fourrage peut, dans certains cas, conduire à la libération dans le système digestif des animaux d'un excès momentané d'ammoniaque avec, pour conséquence, un risque d'intoxication de ceux-ci.

On a donc été amené, pour pallier ces défauts, à remplacer l'urée par des dérivés de celle-ci dont l'hydrolyse, plus lente, permet de libérer l'ammoniaque de manière progressive. Les composés de l'urée ayant des propriétés particulièrement favorables pour une telle utilisation sont ses dérivés glycosidiques. En effet, par leur décomposition ces composés libèrent, simultanément avec de l'ammoniaque, les éléments énergétiques (carbohydrates) nécessaires à la vie de l'animal et à la constitution de ses tissus.

Ainsi, le brevet US 2.748.001 (1956) décrit la préparation d'un aliment pour animaux consistant à mélanger (tout en chauffant jusqu'à ~ 80 °C) les ingrédients suivants :

|  |  |
|---|---|
| Mélasse | 50 % ou plus |
| urée | 2-20 % |
| eau | 25-35 % |
| H$_3$PO$_4$ | 0,27-2 % |
| oligo-éléments | traces |
| pH | 4-4,75 |

Bien que la formation de glucosylurée dans un tel mélange ne soit pas indiquée dans le brevet, on peut la tenir pour probable car l'urée présente dans l'aliment est mieux assimilée par les animaux que si elle avait été fournie séparément.

Le brevet russe N° 264.155 (1970) décrit la fabrication d'un aliment pour animaux contenant, par exemple, des glycosyluréides. On obtient cet aliment en hydrolysant par l'acide (HCl ou H$_2$SO$_4$) à chaud des résidus végétaux, mélasse ou amidon, on ajoute de l'urée à raison de 3 moles par mole de sucre et on chauffe 45-50 h à 55-65 °C. L'acide est ensuite neutralisé à la soude. Un des aliments ainsi fabriqué contient 14,2 % de glucosylurée, 10 % d'urée et 27,4 % de sucres.

Le brevet russe N° 302.093, semblable au précédent, indique que la formation de l'aliment (qui contient les glycosylurées suivantes : mannosylurée, xylosylurée et glucosylurée) est accélérée par adjonction de H$_3$PO$_4$.

Cet acide a également la propriété de fixer l'excès d'urée sous forme de phosphate qui n'a pas les inconvénients alimentaires de l'urée libre.

Le brevet US 3.667.767 décrit en détail l'effet retard sur la libération de l'ammoniaque procuré par l'utilisation, dans les fourrages, des composés d'urée et de saccharides et divulgue un aliment pour animaux et un procédé pour sa préparation. Dans ce procédé on chauffe, au-dessus de 65 °C et à un pH inférieur à 4, (H$_2$SO$_4$ ou H$_3$PO$_4$) un mélange environ 1 : 1 de mélasse et d'urée suffisamment longtemps pour que le taux de conversion de « l'équivalent protéine » total en « équivalent protéine » non décomposable par l'uréase dépasse 20 %.

Cette référence indique encore, mais sans les nommer, que des acides autres que H$_3$PO$_4$ ou H$_2$SO$_4$ sont utilisables dans ce procédé.

Le brevet FR 2.265.286 (75/09723) (1975, priorité 4. 1974) divulgue un aliment pour animaux

**0 006 856**

contenant un mélange réactionnel d'urée, de mélasse et d'acide, au moins 51 % de ce dernier consistant en HCl.

Tous les produits alimentaires issus de l'art antérieur précité, dans lesquels les glycosylurées se présentent sous forme de mélasse ou solutions brutes, ont la faculté de permettre une assimilation de l'urée (des glycosylurées) plus favorable que ne serait celle d'un équivalent d'urée fournie, dans le fourrage, à l'état non lié. Cependant, il n'est fait état nulle part que de tels produits aient, simultanément, la propriété de faciliter et d'augmenter l'assimilation des carbohydrates éventuellement présents dans cet aliment.


## Exposé de l'invention

L'aliment suivant l'invention est caractérisé par le fait que le dérivé d'urée est une glucosylurée solide purifiée à au moins 90 % et dont le total d'urée et de glucose non liés ne dépasse pas 10 %.

On a constaté avec surprise que l'incorporation d'une telle glucosylurée dans un fourrage à base de carbohydrates avait non seulement la faculté de permettre une assimilation favorable de l'azote de l'urée liée mais améliorait et augmentait notablement l'assimilation, par les ruminants, de la fraction carbohydrate de cet aliment. *On remarquera, à ce sujet, que, dans le brevet USP 2.687.354, il est indiqué qu'en présence dans l'estomac des ruminants, de composés azotés, tels que l'urée, la guanidine, le formamide, etc... l'efficacité de la digestion par ceux-ci des matières cellulosiques augmente et qu'un effet similaire est fourni par des condensats urée-aldéhydes. Ce brevet ne fait, cependant, pas mention de fourrages contenant de la glucosylurée purifiée ni de l'effet de celle-ci sur la digestibilité des carbohydrates.*


## Meilleures manières de réaliser l'invention

Comme carbohydrates, l'aliment peut comprendre des amidons comme par exemple du maïs, de l'orge ou d'autres céréales, de la fécule, etc... en proportions avantageusement comprises entre environ 30 et 70 %. Il peut comprendre d'environ 20 à 50 % de produits cellulosiques comme la paille, le foin, les fanes de végétaux, les tourteaux, etc...

Le reste de l'aliment comprend la glucosylurée solide en quantité approximativement 2 à 10 %, se présentant sous forme de cristaux blancs sans odeur, légèrement doux au goût, et, éventuellement, d'autres adjuvants tels que eau, liants, premix, graisses, produits aromatisants, etc... De tels adjuvants sont décrits en détail dans les publications de l'art antérieur (voir par exemple USP 3.677.767 ; 2.748.001 ; 3.753.722 ; 3.684.518 et les textes qui s'y rattachent).

Il est évident que les compositions données ci-dessus ne sont mentionnées qu'à titre indicatif et que les concentrations respectives des ingrédients peuvent sortir, en cas de nécessité, des limites sus-indiquées.

Le procédé de préparation de l'aliment suivant l'invention qui consiste à mélanger ensemble ses différents constituants précités avec la glucosylurée solide est caractérisé par le fait qu'on obtient celle-ci de la manière suivante : on chauffe un équivalent de glucose avec un équivalent d'urée à une température comprise entre environ 60 et 70° en présence d'un acide minéral comme déshydratant et on ajoute environ 30 à 50 % d'eau par rapport au poids total du mélange, ce qui provoque ensuite, par concentration et refroidissement, la cristallisation de la glucosylurée.

Des acides tels que $H_2SO_4$, $H_3PO_4$ ou d'autres acides forts peuvent être utilisés dans ce procédé.

Ce procédé est à distinguer de celui du brevet US 2.612.497 qui mentionne la préparation de glucosylurées à partir de glucose, galactose, arabinose, mannose, fructose et de précurseurs d'hexoses, notamment de polysaccharides et de disaccharides comme le saccharose, le maltose et le lactose. Il décrit plus particulièrement l'obtention de glucosylurée et glucosylthiourée en utilisant, comme source de carbohydrate, soit du glucose pur, soit du sucre de canne et, comme agent de condensation, $H_2SO_4$ ou $H_3PO_4$. Les conditions de travail sont : 60-100 °C ; acide, au plus 20 % ; eau, au plus 20 %. Cependant, ce procédé suivant l'art antérieur conduit à la précipitation d'une glucosylurée cireuse et relativement impure (qu'il est nécessaire de purifier ultérieurement) alors que le présent procédé donne un produit dont la pureté est supérieure à 90 % et qui peut être utilisé tel quel comme ingrédient du présent fourrage. *Ainsi, dans l'Exemple 1 de ce brevet US 2.612.497, il est indiqué, par exemple, de chauffer 10 200 g de glucose hydraté avec 3 400 g d'urée, 600 ml d'eau et 165 ml de $H_2SO_4$ concentré environ 9 h à 60°, après quoi on obtient une masse visqueuse brun foncé à laquelle on rajoute juste assez d'eau pour la dissoudre, on neutralise la solution au·$CaCO_3$, on décolore sur charbon actif, puis on évapore la solution de manière que se séparent 9 200 g de glucosylurée suffisamment pure pour qu'il soit facultatif de procéder à une recristallisation.*

*Par ailleurs, il existe une méthode de préparation de glucosylurée purifiée décrite dans « Advances in Carbohydrate Chemistry 13, 218 (1958). Suivant cette méthode on chauffe du glucose et de l'urée dans de l'acide sulfurique dilué pendant sept jours ce qui fournit un premier composé d'addition qu'on décompose, ensuite, en glucosylurée par trois jours d'ébullition dans l'alcool absolu.*

3

Possibilités d'application industrielle

Les exemples qui suivent illustrent l'invention de manière détaillée.

Exemple 1

Préparation d'un aliment et son essai pour nourrir des ruminants

On a préparé un aliment pour animaux (B) suivant l'invention, et un aliment témoin (A) sans glucosylurée, en mélangeant les ingrédients suivants dans un broyeur puis en procédant a une granulation des mélanges dans une machine adéquate.

Les compositions sont les suivantes :

| Ingrédients | Partie en poids | |
|---|---|---|
| | (A) | (B) |
| maïs moulu fin | 54,5 | 54,5 |
| orge | 10,0 | 6,5 |
| paille moulue | 25,0 | 25,0 |
| marc de fruit | 5,0 | 5,0 |
| graisse | 1,5 | 1,5 |
| urée | 1,5 | — |
| glucosylurée | — | 5,0 |
| liant | 1,5 | 1,5 |
| premix | 1,0 | 1,0 |

On remarquera que 5 g de glucosylurée correspond, au point de vue équivalence chimique, à 1.4 g d'urée libre. Ainsi, l'équivalent urée de l'échantillon (B) était donc légèrement inférieur à celui de l'échantillon (A).

Valeur nutritive calculée pour (A) et (B) :

énergie digestible     3,02 Mcal/kg

protéine brute     11,3 %

On a nourri deux groupes de 10 moutons chacun, l'un avec l'aliment (A) et l'autre avec l'aliment (B) en quantité égale, pendant une période de trois semaines et on a constaté que l'augmentation moyenne de poids des bêtes était de 2 % dans le cas de l'aliment (A) et de 3,5 % dans le cas de l'aliment (B) contenant la glucosylurée. La différence en gain de poids, au profit des animaux nourris avec l'aliment (B), était donc de 75 %. On peut comparer ce chiffre à d'autres valeurs, pour des mesures similaires, relevées sur de bœufs nourris avec des fourrages contenant des glucosylurées de l'art antérieur. Ainsi, par exemple, le cas où, avec de la glucosylurée dérivée d'un hydrolyzat d'amidon, le gain en poids était de 20 % par comparaison avec une alimentation sans glucosylurée (voir USP 4.044.156) et le cas où, avec des mélanges dits « mélasse-urées », le gain en poids par rapport à une alimentation contenant de l'urée libre était de 6,7 % et, par rapport à une alimentation contenant des protéines naturelles, de 7,5 % (voir USP 3.667.767).

De plus, pendant la durée de l'essai, on a prélevé à intervalles réguliers sur certains des moutons des échantillons de suc de rumen aux fins d'analyse. Celles-ci ont porté sur la variation de pH et le taux d'ammoniaque, ce qui constitue une mesure directe du taux de dégradation des matières azotées de l'aliment. Les résultats sont consignés aux Tableaux I et II ci-dessous en fonction du temps écoulé après ingestion des aliments.

TABLEAU I

Variation du pH

| temps (min) | (A) | (B) |
|---|---|---|
| 0 | 7,30 | 7,08 |
| 20 | 7,16 | 6,78 |
| 40 | 6,98 | 6,55 |
| 60 | 6,70 | 6,37 |
| 120 | 5,88 | 6,18 |
| 240 | 6,10 | 6,04 |
| 360 | 6,53 | 6,16 |
| 480 | 6,80 | 6,40 |

### TABLEAU II
#### Variation du taux d'ammoniaque

| temps (min) | (A) | (B) |
|---|---|---|
| 0 | 4,37 | 6,43 |
| 20 | 33,72 | 11,88 |
| 40 | 26,54 | 12,02 |
| 60 | 20,80 | 10,32 |
| 120 | 4,05 | 9,53 |
| 240 | 0,93 | 3,33 |

On voit d'après les résultats des Tableaux I et II que la libération d'ammoniaque se fait plus lentement dans le cas de l'aliment (B) contenant la glucosylurée que dans celui de l'aliment (A) contenant de l'urée libre.

Les mesures analytiques ont aussi porté sur la formation, avec le temps de digestion, des acides volatils. Cette mesure est importante car elle traduit directement le taux de dégradation des carbohydrates et en conséquence la faculté d'assimilation de ceux-ci. Les résultats figurent ci-dessous au Tableau III en ce qui concerne l'aliment (B) et au Tableau IV en ce qui concerne le témoin (A).

### TABLEAU III
#### Acides volatils en mM/l : aliment (B)

| Temps après le repas (min) | 0 | 60 | 120 | 240 | 360 | 480 |
|---|---|---|---|---|---|---|
| **Acides** | | | | | | |
| — acétique | 22,72 | 37,44 | 47,02 | 55,14 | 45,71 | 43,09 |
| — propionique | 8,26 | 19,80 | 26,97 | 31,51 | 27,05 | 25,47 |
| — iso-butyrique | 0,71 | 0,49 | 0,52 | 0,49 | 0,46 | 0,38 |
| — butyrique | 4,01 | 9,04 | 12,03 | 14,91 | 8,05 | 7,30 |
| — iso-valérique | 0,91 | 0,50 | 0,37 | 0,59 | 0,49 | 0,57 |
| — valérique | 0,67 | 1,43 | 1,31 | 1,91 | 0,94 | 0,79 |
| — caproïque | 0,24 | 0,28 | 0,45 | 0,61 | 0,35 | 0,20 |
| Total | 37,51 | 68,96 | 88,67 | 105,14 | 83,02 | 77,78 |

### TABLEAU IV
#### Acides volatils en mM/l : aliment (A)

| Temps après le repas (min) | 0 | 60 | 120 | 240 | 360 | 480 |
|---|---|---|---|---|---|---|
| **Acides** | | | | | | |
| — acétique | 16,87 | 36,69 | 45,88 | 39,52 | 35,40 | 32,46 |
| — propionique | 5,82 | 20,55 | 31,29 | 31,18 | 23,55 | 17,41 |
| — iso-butyrique | 0,52 | 0,43 | 0,40 | 0,38 | 0,39 | 0,49 |
| — butyrique | 1,75 | 4,86 | 8,22 | 9,03 | 8,12 | 6,51 |
| — iso-valérique | 0,64 | 0,36 | 0,21 | 0,40 | 0,56 | 0,65 |
| — valérique | 0,46 | 1,06 | 1,35 | 1,43 | 1,08 | 0,74 |
| — caproïque | 0,06 | 0,14 | 0,27 | 0,25 | 0,36 | 0,32 |
| Total | 26,11 | 64,08 | 87,61 | 82,17 | 69,45 | 58,36 |

Les résultats des Tableaux III et IV sont particulièrement surprenants du fait que l'aliment (B) ne contient environ que 2 % de plus d'amidon que l'aliment (A) ; ceci en raison du fait que le glucose de la glucosylurée remplace une partie de l'amidon de l'orge, qui elle ne contient pas que de l'amidon. Il serait donc logique de retrouver à peu près les mêmes quantités d'acides volatils formées dans la digestion de l'aliment (B) et celle de celui contenant l'urée. Or, les résultats ci-dessus montrent que cela n'est pas le cas : la glucosylurée solide conduit à une concentration moyenne d'acides supérieure à celle provenant de l'aliment contenant de l'urée : 84,6 contre 71,3 mM/l respectivement (+ 18,7 %). Il est à remarquer que la production accrue d'acides volatils ne s'effectue pas immédiatement après le repas, mais seulement 2-3 heures après. On est donc amené à postuler que la glucosylurée, ou une partie de ses composants, a

5

une action stimulatrice sur la dégradation de carbohydrates autres que l'amidon. A des degrés divers. les concentrations moyennes des acides acétique, propionique, butyrique sont augmentées, l'effet est le plus important sur l'acide acétique (45,3 contre 36,6 mM/l soit + 23, 1/2 %).

La glucosylurée utilisée dans le présent exemple avait les constantes physiques suivantes :

| | |
|---|---|
| Humidité | 1,2 % |
| Azote total | 13,55 % |
| Urée libre | 3,15 % (titrée par uréase) |
| Glucose libre | 2,3 % (titrée par glucose oxidase) |
| Pureté | 94,55 % |
| Equivalent urée du total | 25,6 % environ |
| Equivalent azoté | 13,5 % calculé sur le poids sec |
| Equivalent protéique (6.25 × N %) | 6.25 × 13.55 = 84.69/100 g |
| Point de fusion ($\alpha$) | 198 °C (avec décomposition) |
| Cristaux blancs, sans odeur, légèrement doux au goût (l'urée seule est amère) | −16° dans $H_2O$, c = 2 % |

## Exemple 2

### Préparation de la glucosylurée cristallisée

Dans un bécher de 6 litres, thermostatisé à 60 °C et muni d'un puissant agitateur, on introduit 204 g de glucose monohydrate et 60 ml d'eau.

On agite le mélange pendant 3 min et additionne ensuite 680 g d'urée. Au bout de 10 min, la masse fond et donne un sirop épais. La température du sirop est de 60 °C. On ajoute alors toujours sous agitation 1 836 g de glucose monohydrate par portions de 200 g environ. Après 90 min (temps nécessaire à ces additions) on ajoute 33 ml d'acide sulfurique à 98 % dans 60 ml d'eau.

On maintient le mélange sous agitation à 68 °C pendant 8 heures, puis à la masse visqueuse on ajoute 2 litres d'eau. Le pH du mélange en solution est égal à 1. On ajoute alors 67 g de $CaCO_3$ et on laisse une nuit sous agitation en présence de charbon actif (15 g) en laissant revenir à température ambiante. Le lendemain on filtre sur filtre/papier pour sirop, puis on concentre sous pression réduite jusqu'à obtention de cristaux abondants dans un sirop. Ces cristaux (850 g) sont filtrés sur büchner avec papier filtre. puis séchés sous vide en présence de $P_2O_5$.

Cristaux jaune clair F-190 °C (dec.).

On concentre la liqueur obtenue après séparation du premier jet jusqu'à nouvelle cristallisation et essore à nouveau 1 100 g de cristaux de coloration légèrement plus foncée. F 180-200° (dec.) après lavage à l'éthanol bouillant et séchage sur $P_2O_5$. La pureté de cette deuxième récolte dépassait 90 % et on a pu l'utiliser sans autre pour préparer le fourrage de l'invention.

| Analyse | 1$^{er}$ jet | 2$^e$ jet | (après recristallisation dans $H_2O$ ;) |
|---|---|---|---|
| N total | 12,9 | 12,5 | |
| urée libre | 3,1 | 2,2 | |
| glucose libre | 2,3 | 1,3 | |
| pureté | 94,6 | 96,5 | |

## Revendications

1. Aliment pour bétail à base d'hydrates de carbone et d'un dérivé d'urée, caractérisé par le fait que celui-ci est une glucosylurée cristallisée purifiée à au moins 90 % et dont le total d'urée et de glucose non chimiquement liés ne dépasse pas 10 % en poids.

2. Aliment suivant la revendication 1, caractérisé par le fait que les hydrates de carbone sont, d'une part, l'amidon et, d'autre part, la cellulose et les hémicelluloses, en quantités respectives de 30 à 70 % et de 20 à 50 % en poids de l'aliment.

3. Aliment suivant la revendication 2, caractérisé par le fait qu'il contient, en outre, au moins l'un des ingrédients suivants : eau, liants, premix, graisse, aromates.

4. Procédé de préparation de l'aliment suivant la revendication 1 lequel consiste à mélanger ensemble ses différents constituants avec la glucosylurée, caractérisé par le fait que pour obtenir celle-ci on chauffe à environ 60-70 °C un équivalent de glucose avec un équivalent d'urée en présence d'un acide fort, tel que l'acide sulfurique, comme déshydratant, qu'on dilue avec de l'eau de façon que, par concentration et refroidissement. il se dépose par cristallisation de la glucosylurée de pureté supérieure à 90 %, et qu'on récolte celle-ci.

5. Utilisation de la glucosylurée cristallisée comme ingrédient d'aliment pour ruminants contenant de la cellulose et de l'hémicellulose en tant que stimulateur de la digestion et de l'assimilation de celles-ci par ces animaux.

**Claims**

1. Feedstuff for livestock based on carbohydrates and a urea derivative, characterized by the fact that the latter is a crystallized glucosylurea the purity of which is at least 90 % and the total chemically unbound urea and glucose of which does not exceed 10 % by weight.

2. Feedstuff according to claim 1, characterized by the fact that the carbohydrates thereof comprise on one part starches and on another part cellulose and hemicelluloses in quantity of, respectively, 30 to 70 % and 20 to 50 % by weight of the feedstuff.

3. Feedstuff according to claim 2, characterized by the fact that it further comprises at least one of the following additives : water, binders, premixes, fats, flavors.

4. Process for the preparation of the feedstuff according to claim 1 which consists in mixing together the various constituents thereof with glucosylurea, characterized by the fact that for obtaining the latter one heats to about 60-70 °C one equivalent of glucose with one equivalent of urea under stirring in the presence of a strong acid such as $H_2SO_4$ as a dehydrating agent, one dilutes with about 30-50 % by weight of water in a manner such that by concentrating and cooling, there will deposit by crystallization glucosylurea of purity over 90 %, and one collects such crystals.

5. The use of crystallized glucosylurea as an ingredient of a feedstuff for ruminant containing cellulose and hemicellulose as a stimulant for the digestion and the assimilation thereof by the animals.

**Ansprüche**

1. Viehfuttermittel auf der Basis von Kohlehydraten und einem Harnstoffderivat, dadurch gekennzeichnet, daß dieses ein kristallisierter, mindestens zu 90 % gereinigter Glucosylharnstoff ist und dessen Gesamtbestandteil an nicht chemisch gebundenem Harnstoff und Glucose 10 Gew% nicht übersteigt.

2. Futtermittel nach Anspruch 1, dadurch gekennzeichnet, daß die Kohlehydrate einerseits die Stärke, andererseits die Zellulose und die Hemizellulosen in jeweiligen Mengen von 30 bis 70 Gew% und von 20 bis 50 Gew% des Futtermittels sind.

3. Futtermittel nach Anspruch 2, dadurch gekennzeichnet, daß es außerdem mindestens den einen der folgenden Bestandteile enthält: Wasser, Bindemittel, Premix, Fett, aromatische Substanzen.

4. Verfahren zur Herstellung eines Futtermittels nach Anspruch 1, das im Vermischen seiner verschiedenen Bestandteile mit dem Glucosylharnstoff besteht, dadurch gekennzeichnet, daß man, um diesen zu erhalten, ein Glucoseäquivalent mit einem Harnstoffäquivalent in Anwesenheit einer starken Säure wie Schwefelsäure als Entwässerungsmittel auf etwa 60 bis 70°C erhitzt, daß man mit Wasser verdünnt, so daß er sich infolge Konzentration und Abkühlung durch Kristallisierung des Glucosylharnstoffs mit einer Reinheit über 90 % ablagert, und daß man diesen dann gewinnt.

5. Verwendung des kristallisierten Glucosylharnstoffs alls Futtermittelbestandteil für Wiederkäuer, der Zellulose und Hemizellulose als Stimulans für die Verdauung und die Assimilierung dieser durch diese Tiere enthält.